# DEMANDE DE BREVET EUROPEEN

(11) **EP 1 568 711 A1**
(43) Date de publication de la demande: **31.08.2005**
(21) Numéro de dépôt: 05290183.2
(22) Date de dépôt: 27.01.2005
(51) Int. Cl.: C07K 16/10, C07K 16/04, A61K 39/395

(54) **Composition comprenant des immunoglobulines spécifiques du rotavirus**

(30) Priorité: 27.01.2004 FR 0400756
(71) Demandeur: Prevendia, 92140 Clamart (FR)
(72) Inventeur: Petillot, Frédéric, 92140 Clamart (FR); Djiane, Jean, 91370 Verrières le Buisson (FR)
(74) Mandataire: Breese, Pierre

(57) **Abrégé**

La présente invention concerne une composition à administration orale chez l'homme comprenant des immunoglobulines spécifiques du rotavirus produites chez un mammifère immunisé avec des pseudo-particules virales. La présente invention concerne également l'utilisation de ladite composition comme additif alimentaire et un procédé de préparation de ladite composition.

## Description

La présente invention appartient au domaine de la prévention et/ou du traitement des infections virales et, plus particulièrement, des rotaviroses. La présente invention concerne une composition à administration orale visant à fournir une protection passive préventive ou curative contre les virus et, plus particulièrement, contre les rotavirus.

Les diarrhées font partie des maladies les plus fréquemment rencontrées chez l'homme et notamment chez l'enfant de moins de 5 ans (Snyder et Merson, 1982, Bull WHO, vol. 60, pages 605-631). Les agents responsables de ces maladies sont des bactéries, des parasites ou des virus tels que les rotavirus.

Les infections à rotavirus sont un problème mondial. A travers le monde, 95 % des enfants sont infectés avant l'âge de 5 ans. Le pic d'infection a lieu entre 4 et 36 mois d'âge. L'infection des adultes est possible, mais elle est le plus souvent asymptomatique. Cependant, elle peut devenir clinique chez les parents d'enfants malades, les immunodéprimés, les voyageurs dans les pays en voie de développement (PVD) et les personnes âgées. Chez les enfants immunodéprimés, les gastro-entérites à rotavirus évoluent de façon chronique (Gault et Garbarg-Chenon, 2000, Revue française des laboratoires, vol. 320, pages 55-62). Aux Etats-Unis, des pics de diarrhées ont été observés à des saisons différentes en fonction des régions. En France, le pic a lieu en hiver entre novembre et décembre (Gendrel et al., 1999, Archives de pédiatrie, vol. 6, pages 739-739). Cette saisonnalité est moins observée dans les PVD où le rotavirus est présent tout au long de l'année.

Aux Etats-Unis, 2,7 millions d'enfants de moins de 5 ans sont infectés par le rotavirus chaque année. Ces diarrhées sont rarement mortelles dans les pays développés. Aux Etats-Unis, 20 à 40 morts annuelles ont été imputées au rotavirus selon une étude de 1996 (Glass et al., 1996, Journal of infectious diseases, vol. 174, pages S5-S11). Une étude de 1990 estimait ce nombre entre 75 et 125. Ces diarrhées ont conduit à 50 000 hospitalisations, c'est-à-dire 3 à 4 % des hospitalisations d'enfants de moins de 5 ans (Smith et al., 1995, Pediatrics, vol. 96, pages 609-615). Ces hospitalisations et les visites chez le médecin génèrent un coût direct de 274 millions de dollars et un coût indirect de 1 milliard de dollars. Une enquête rétrospective réalisée en 1999 en France concluait que 20 à 50 % des diarrhées aiguës nécessitant une hospitalisation pouvaient être imputées au rotavirus (Desenclos et al., 1999, Acta pediatrica, vol. Suppl. 426, pages 42-47.).

Dans les PVD, le nombre annuel de décès dus aux diarrhées est estimé à 3 millions (Glass et al., 1994, *Science,* vol. 265, pages 1389-1391). Le rotavirus serait responsable de 800 000 de ces décès (soit près de 25%), ce qui en fait la première ou deuxième cause de mortalité des enfants de moins de 5 ans dans ces pays. De plus, le rotavirus est responsable de 20 à 40 % des hospitalisations pour cause de diarrhées dans les PVD (Glass et al., 1997, Nature Medecine, vol. 3, pages 1324-1325).

En Afrique du sud, le quart des hospitalisations pour diarrhée est dû au rotavirus. Chez les enfants de moins de 12 mois, il est impliqué dans 75% des cas (Steele et al., 2002, Vaccine, vol. 3563, pages 1-7). Au Brésil, le rotavirus serait impliqué dans 12 à 42 % des cas diarrhées sévères ayant conduit à une hospitalisation.

Les rotaviroses sont aussi des maladies nosocomiales, fréquentes dans certains services de pédiatrie. Dans une enquête réalisée au service hospitalier de Versailles, 4,3% des enfants admis avec un transit normal ont développé une gastro-entérite nosocomiale (Pina et al., 2000, Archives de pédiatrie, vol. 7, pages 1050-1058). Par ailleurs, une enquête rétrospective réalisée à Paris en 1998 a montré que le rotavirus en tant que maladie nosocomiale est souvent associé au VRS (pour " Virus Respiratoire Syncytial "). A Paris, le pic de diarrhées coïncide avec celui des bronchiolites, ce qui conduit à un encombrement des services de pédiatrie. Ce surnombre favorise la transmission des diarrhées nosocomiales pour les enfants hospitalisés pour une cause autre que la diarrhée (Gendrel et al., 1999, Archives de pédiatrie, vol. 6, pages 739-739). Quelques études font référence à des épidémies de gastro-entérites au sein de services de gériatrie (Gault et Garbarg-Chenon, 2000, Revue française des laboratoires, vol. 320, pages 55-62).

Les rotavirus, virus de la famille des Réoviridae, découverts en 1973 par Bishop et al. (Lancet, vol. i, pages 1281-1283) sont des virus non enveloppés de 70 nm de diamètre et dont la capside présente une symétrie isosaèdrique. On connaît 7 groupes de rotavirus référencés de A à G. Il existe une variation spacio-temporelle des souches.

Le génome des rotavirus est composé de 11 segments d'ARN double brin (Mattion et al., 1994, The rotavirus proteins. Dans : Marcel Dekker ed., *Viral infections of the gastrointestinal tract.* Kapikian A.Z., New York, pages 169-250). Chaque segment code pour une protéine. Il y a 7 protéines structurales dénommées VP 1 à VP 7 et 6 protéines non structurales dénommées NSP 1 à NSP 6. La capside est composée de trois couches protéiques concentriques. La couche interne (ou core) renferme le génome. Elle est formée par une protéine majoritaire, VP 2 (94 kD), et par deux protéines minoritaires, VP 1 (125 kD) et VP 3 (88 kD). La couche intermédiaire est formée par la protéine VP 6 (41 kD). VP 6, protéine la plus abondante, porte la spécificité de groupe et sert, donc, au diagnostic. La couche externe (ou capside) est formée par la protéine VP 4 (88 kD) et la glycoprotéine VP 7 (38 kD) qui induisent toutes deux des anticorps neutralisants (Mattion et al., 1994, The rotavirus proteins. Dans : Marcel Dekker ed., *Viral infections of the gastrointestinal tract.* Kapikian A.Z., New York, pages 169-250). VP 4 est impliquée dans l'attachement du virus aux récepteurs cellulaires (Gault et Garbarg-Chenon, 2000, Revue française des laboratoires, vol. 320, pages 55-62). Il existe également une protéine appelée VP 8 qui correspond à un sous-ensemble de VP 4 et qui induit aussi des anticorps neutralisants.

La contamination par les rotavirus se fait par voie oro-fécale. La résistance des rotavirus et la grande quantité de virus excrétés (10¹⁰ particules virales par gramme de selles) favorisent la transmission entre individus directement ou indirectement par l'intermédiaire de surfaces ou d'objets contaminés.

Le rotavirus se multiplie dans les entérocytes matures des villosités de l'intestin grêle. La pénétration du rotavirus dans les cellules met en jeu les protéines VP 4 et VP 7. Après la transcription et la réplication du génome viral, les ARN viraux sont encapsidés et les virions se forment. La lyse des cellules infectées libère les particules virales.

La multiplication virale entraîne une vacuolisation, une lyse et une desquamation des entérocytes. Cela conduit à une atrophie des villosités intestinales accompagnée d'une hypertrophie et d'une augmentation des cellules sécrétoires des cryptes. Quand il n'y a plus d'entérocyte différencié sur les villosités de l'intestin grêle, les nutriments non digérés fermentent augmentant la pression osmotique intraluminale. Il s'agit d'une diarrhée de malabsorption. L'entérotoxine NSP4 induit une sécrétion de Cl⁻, ce qui conduit à une diarrhée sécrétoire (Pothier et Kohli, 2002, Reoviridae. Dans : MAMMETTE A. ed., *Virologie médicale.* Presses Universitaires de Lyon, Lyon, pages 433-445).

La guérison survient dans les 7 jours (Estes, 2001, Rotavirus and their replication. Dans : FIELDS B.N., KNIPE D.M., HOWLEY P.M. et GRIFFIN D.E. ed., *Fields's virology*. Lippincott Williams & Wilkins, Philadelphia, pages 1747-1785). La diarrhée peut être modérée ou sévère et entraîner la mort par déshydratation. Des vomissements sont très fréquemment observés.

A l'heure actuelle, il n'existe pas de traitement spécifique des diarrhées à rotavirus. Le traitement est symptomatique. Il consiste à restaurer la balance hydrique et électrolytique puis à maintenir l'hydratation jusqu'à la fin de la diarrhée. La réhydratation peut être faite par voie orale avec de bons résultats. Une hospitalisation et une réhydratation par voie veineuse sont nécessaires dans les cas sévères (Espgan, 1997, *Journal of pediatric gastroenterology and nutrition,* vol. 24, pages 619-620).

Dans le cadre du traitement anti-rotavirus, des vaccins à base de pseudo particules virales (VLP pour " Virus Like Particles ") ont été proposés. Un de ces vaccins a notamment été commercialisé aux USA sous le nom Rotashield™ en 1998. Toutefois, l'observation d'effets secondaires chez l'homme du type invagination intestinale a suspendu l'utilisation de tels vaccins (Gay et al., 1999, Lancet, vol. 354, page 956). A ce problème technique, s'ajoutent les limites que présente la vaccination chez l'enfant. En effet, les anticorps maternels interfèrent avec la réponse vaccinale ce qui réduit l'efficacité du vaccin. Les nouveau-nés développent rarement des diarrhées à rotavirus grâce à la protection conférée par les anticorps maternels. Cependant, cette protection maternelle peut ne pas être suffisante. Dans ce cas, le recours à la vaccination ne permettra pas une protection immédiate à cause du délai d'apparition des anticorps après la réalisation de l'injection vaccinale.

Il existe donc un réel besoin d'un traitement visant à prévenir et/ou à réduire les infections dues aux rotavirus. La solution technique proposée dans le cadre de la présente invention est l'immunisation passive spécifique anti-rotavirus.

Plus particulièrement, la présente invention concerne une composition à administration orale chez l'homme comprenant des immunoglobulines spécifiques du rotavirus produites chez un mammifère immunisé avec des pseudo particules virales.

Par " pseudo particule virale ", on entend dans le cadre de la présente invention tout assemblage de protéines rétrovirales dépourvues de génome. Cet assemblage est une particule inerte ne présentant aucun pouvoir infectieux et correspondant en partie ou en totalité à la structure protéique du rotavirus.

Pour le rotavirus, différentes VLP peuvent être obtenues : VLP 2/6 (formées des protéines structurales VP2 et VP6), VLP 2/4/6, VLP 2/6/7 et VLP 2/4/6/7 (Crawford et al., 1994, *Journal of Virology,* vol. 68, pages 5945-5952). Les VLP peuvent être hétérologues (formées de protéines issues de virus d'espèces différentes) ou homologues (formées de protéines issues du même virus).

L'homme du métier connaît un procédé pour obtenir des VLP utilisant des systèmes d'expression avec baculovirus et cellules d'insectes. Ainsi, les gènes codant les protéines structurales du rotavirus (VP) sont introduits dans le génome de baculovirus. Des cellules d'insectes sont mises en présence de ces baculovirus recombinants. Les gènes codant pour les protéines structurales de rotavirus sont alors exprimés dans les cellules d'insectes. Les VP ainsi synthétisées s'assemblent spontanément en particules virales que l'on nomme VLP. La structure des VLP obtenues dépend des gènes que l'on introduit dans la cellule d'insecte via le baculovirus recombinant. La VLP peut être formée d'une seule couche protéique lors de la seule expression des VP 2. De deux couches protéiques lors de l'expression de VP2 et VP6 ou de VP 2, VP 4, VP 6 et de trois couches lors de l'expression de VP 2, VP 6 et VP 7 ou de VP 2, VP4, VP6, VP7 (Crawford et al., 1994, *Journal of Virology,* vol. 68, pages 5945-5952).

Les VLP 2/6 utilisées dans les exemples ci-après sont obtenues en co-infectant les cellules de *Spodoptera frugiperda 9* avec les baculovirus recombinants BacRF2A et pAC461/SA11-6. BacRF2A, obtenu à partir de la souche bovine RF, exprime la protéine VP 2 bovine, et pAC461/SA11-6, obtenu à partir de la souche simienne SA11, exprime la protéine VP 6 (Labbé et al., 1991, *Journal of Virology,* vol. 65, pages 2946-2952). Ces VLP 2/6 sont donc formées de deux couches protéiques : la capside interne et de la couche protéique intermédiaire.

L'homme du métier connaît également d'autres systèmes d'expression qui pourront être mis en oeuvre dans le cadre de la présente invention pour produire des pseudo particules virales. A titre d'exemples, ces systèmes d'expression peuvent utiliser des levures, des cellules animales ou des cellules végétales transfectées avec au moins une séquence d'acide nucléique codant une protéine structurale du rotavirus ou une partie de cette protéine structurale et capables d'exprimer la protéine correspondante. Les systèmes de transfection mettant en oeuvre des vecteurs d'expression seront choisis en fonction de l'hôte cellulaire dans lequel l'expression protéique doit avoir lieu.

Ainsi, les vecteurs d'expression utilisables pour transfecter l'hôte cellulaire comprendront, en plus de la séquence nucléique codant au moins une protéine structurale du rotavirus ou une partie de cette protéine structurale, au moins un élément choisi dans le groupe des promoteurs constitutifs, des promoteurs inductibles, des éléments terminateurs, des éléments régulateurs de la transcription, de la traduction et de la maturation des peptides et des éléments permettant la sélection dans l'organisme hôte (gène de sélection ou gène de résistance à un antibiotique). Ces différents éléments sont liés entre eux et avec la séquence nucléique codant au moins une protéine structurale du rotavirus ou une partie de cette protéine structurale de façon opérationnelle, de façon à ce que le fonctionnement d'un de ces éléments soit affecté par celui d'un autre. Les vecteurs d'expression utilisables dans le cadre de la présente invention sont avantageusement choisis parmi les plasmides, les cosmides, les bactériophages et les virus en particulier les baculovirus.

Les séquences des molécules d'acide nucléique codant une protéine structurale du rotavirus utilisables pour obtenir des VLP sont des données de l'état de la technique et peuvent notamment être obtenues dans Genbank. A titre d'exemples, quelques références Genbank sont données ci-après :
- AY 238390 : ADNc du gène de VP2 du rotavirus B humain souche Bang373 ;
- AY 238389 : ADNc du gène de VP6 du rotavirus B humain souche Bang373 ;
- AY 238388 : ADNc du gène de VP4 du rotavirus B humain souche Bang373 ;
- AY 238385 : ADNc du gène de VP7 du rotavirus B humain souche Bang373 ;
- AB 077057 : ADNc du gène de VP7 du rotavirus bovin ;
- D 003226 : ADNc du gène de VP6 du rotavirus porcin.

Il est évident, pour l'homme du métier, que des séquences comprenant une ou plusieurs modifications par rapport à ces séquences de l'état de la technique sans affecter leur rôle de protéine structurale peuvent être utilisées dans le cadre de la présente invention.

Par conséquent, les pseudo particules virales utilisées pour immuniser le mammifère dans le cadre de la présente invention sont soit des pseudo particules virales homologues, soit des pseudo particules virales hétérologues.

De façon préférée, les pseudo particules virales utilisées pour immuniser le mammifère dans le cadre de la présente invention sont des pseudo particules virales contenant plusieurs protéines structurales différentes. Tout particulièrement, une de ces protéines structurales est la VP2. Ainsi, les particules pseudo virales sont choisies dans le groupe comprenant VLP 2/6, VLP 2/7, VLP 2/4/6, VLP 2/6/7, VLP 2/4/6/7 et VLP 2/8/6/7.

De façon préférée, les immunoglobulines présentes dans la composition de l'invention sont des immunoglobulines A.

Lesdites immunoglobulines et, tout particulièrement, lesdites immunoglobulines A sont obtenues à partir du colostrum produit par ledit mammifère. Ainsi, lesdites immunoglobulines et, tout particulièrement, les immunoglobulines A sont présentes, dans la composition de l'invention, sous la forme d'une fraction semi-purifiée du colostrum. D'un point de vue légal, le colostrum est la sécrétion lactée des premiers jours qui suivent la mise bas. Chez la chèvre, il s'agit de la sécrétion lactée des 7 premiers jours après la mise bas. Le colostrum est caractérisé par une très grande richesse en protéines dérivées du sang que sont les immunoglobulines, albumine, 2-macroglobuline et transferrine. Les immunoglobulines représentent 70 à 80 % des protéines du colostrum du premier jour alors que dans le lait, elles ne représentent plus que 1 à 2 % des protéines (Korhonen et al., 2000, British Journal of Nutrition, vol. 84, pages S75-S80).

Des travaux antérieurs ont montré que l'administration journalière à un enfant de 50 ml de colostrum bovin obtenu à partir d'un animal non-spécifiquement immunisé permettait d'obtenir une amélioration de l'état de santé dudit enfant. Les performances obtenues avec les chèvres immunisées spécifiquement avec des VLP correspondent à une concentration en immunoglobulines spécifiques anti-VLP dans le colostrum desdites chèvres 10 fois supérieures à la concentration en immunoglobulines dans le colostrum bovin initialement testé. Ainsi, dans le cadre de la présente invention, il est raisonnable d'envisager d'administrer à l'homme une composition contenant une quantité d'immunoglobulines présentes dans entre 1 et 100 ml de colostrum et, de préférence, présentes dans entre 5 et 10 ml de colostrum.

De façon avantageuse, le mammifère immunisé par les pseudo particules virales est un mammifère non humain et est choisi dans le groupe constitué par la vache, la brebis, la chèvre, la lapine, la truie, etc... . De façon tout particulièrement préférée, ledit mammifère est un ruminant laitier et, de préférence, une chèvre.

De plus, la composition objet de la présente invention peut comprendre en plus desdites immunoglobulines tout véhicule acceptable. Par " véhicule acceptable ", on entend dans le cadre de la présente invention toute substance qui est ajoutée auxdites immunoglobulines pour favoriser leur transport, éviter leur dégradation substantielle dans la composition et préserver leurs propriétés biologiques.

Dans le cadre de la présente invention, la composition telle que définie précédemment est utile pour la protection passive contre les pathologies induites par les rotavirus.

La présente invention concerne également l'utilisation d'une composition telle que définie précédemment en tant que complément alimentaire.

Enfin, la présente invention concerne un procédé de préparation d'une composition telle que définie précédemment comprenant les étapes suivantes :
a) préparation des pseudo particules virales,
b) immunisation d'un mammifère avec les pseudo particules virales obtenues à l'étape (a),
c) récupération du colostrum produit par ledit mammifère,
d) éventuellement concentration des immunoglobulines contenues dans le colostrum récupéré à l'étape (c).

Les différentes formes de mise en oeuvre de l'étape (a) du procédé objet de la présente invention ont été décrites précédemment. Ainsi, tout moyen connu de l'homme du métier pour produire des pseudo particules virales avec des systèmes d'expression utilisant des levures, des cellules animales comme des cellules d'insectes ou des cellules de plantes sont utilisables dans le cadre de la présente invention.

Les pseudo particules virales ainsi produites sont utilisées pour immuniser un mammifère, ce qui correspond à l'étape (b) du procédé objet de l'invention. La partie expérimentale ci-après décrit une immunisation par voie intramusculaire avec de l'adjuvant de Freund incomplet. D'autres voies d'immunisation peuvent également être mises en oeuvre dans le cadre de la présente invention comme la voie intra-nasale, la voie intra-vaginale ou per os.

L'adjuvant dans lequel les pseudo particules virales sont mises avant l'immunisation dépendra de la voie d'immunisation choisie. Parmi les adjuvants utilisables dans le cadre de l'invention, on peut citer les microsphères d'alginate, les ISCOM's (structures formées de saponine de Quillaja et de cholesterol) décrites par Hu et al., 2001 (Advanced drug delivery reviews, vol. 51, pages 149-159), la toxine thermolabile d'E. coli atténuée (LT-R192R), la toxine cholérique, les émulsions "eau dans huile" (ex : montanide ISA 50, les émulsions "eau dans huile dans eau" (ex : montanide ISA 206) et les émulsions "huile dans eau" (ex : montanide ISA 25), etc... .

La partie expérimentale ci-après décrit l'immunisation de chèvres avec 50 µg de VLP 2/6 suivie d'un rappel avec 22 µg de VLP 2/6. Par conséquent, dans le cadre du procédé de l'invention, la quantité de pseudo particules virales à utiliser pour immuniser le mammifère est comprise entre 2 et 500 µg et, de préférence de l'ordre de 50 µg. Il est clair que des variations desdites quantités sont envisageables en fonction du mammifère choisi et en fonction du type de pseudo particules virales. De plus, l'immunisation de l'étape (b) du procédé objet de la présente invention peut être une immunisation en une seule étape ou une immunisation à plusieurs étapes (i.e. une première immunisation suivie d'un ou plusieurs rappels).

Par conséquent, l'immunisation de l'étape (b) est une immunisation par voie intramusculaire, par voie intra-nasale, par voie intra-vaginale ou per os.

Dans le cadre du procédé objet de la présente invention, l'éventuelle étape (d) de concentration des immunoglobulines contenues dans le colostrum met en oeuvre des techniques classiques de concentration et/ou de purification connues de l'homme du métier. La partie expérimentale ci-après compare notamment deux techniques que sont la lyophilisation et l'atomisation. Lors de cette étape de concentration, d'autres étapes préliminaires peuvent être mises en oeuvre comme un écrémage du colostrum, une précipitation des caséines, des centrifugations, des filtrations et/ou des ultrafiltrations. Sur la base des protocoles et notamment des protocoles de dosages ELISA décrits dans la partie expérimentale ci-après, l'homme du métier sera capable, après l'éventuelle étape (d), de vérifier la concentration des immunoglobulines anti-rotavirus et de vérifier l'activité anti-rotavirus de ces immunoglobulines.

Ainsi, la concentration des immunoglobulines à l'étape (d) du procédé objet de la présente invention comprend au moins une étape choisie dans le groupe constitué par un écrémage du colostrum, une précipitation des caséines, des centrifugations, des filtrations, des ultrafiltrations, une lyophilisation et une atomisation.

D'autres avantages et caractéristiques de l'invention apparaîtront à la lecture des exemples qui suivent concernant l'immunisation de chèvres avec la particule pseudo-virale VLP2/6 et VLP2/8/6/7, la titration des immunoglobulines dans le colostrum produit par les chèvres ainsi immunisées ainsi que l'extraction des immunoglobulines colostrales anti-rotavirus.

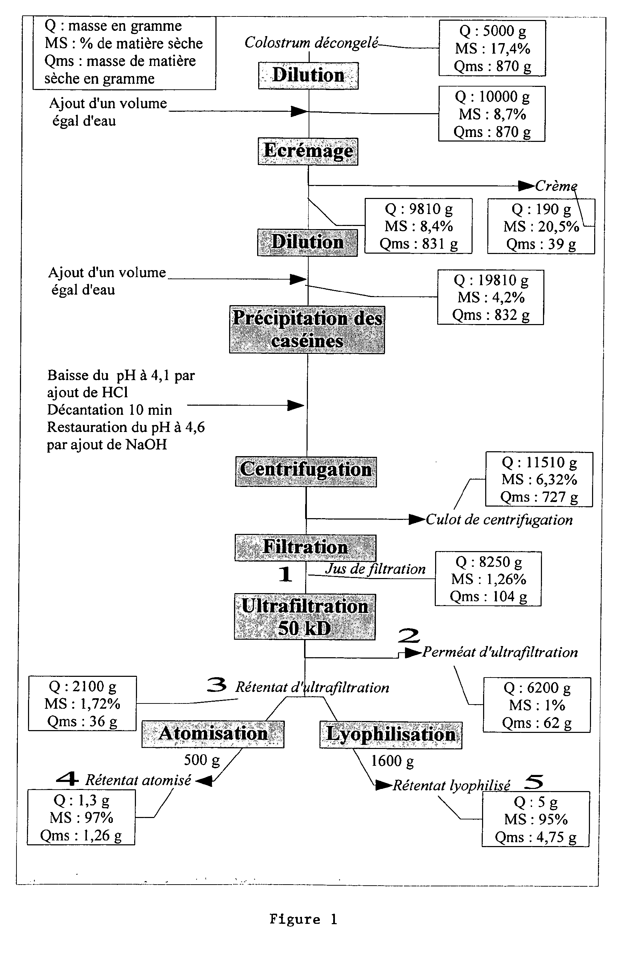

Ces exemples sont donnés à titre illustratif et ne sauraient être interprétés comme limitant la portée de l'invention. Ils font référence à la figure 1 qui présente le procédé de purification des immunoglobulines colostrales et le diagramme de masse.

### I. IMMUNISATION DES CHEVRES PAR DES PARTICULES PSEUDO-VIRALES.

### I.1 IMMUNISATION DES CHEVRES PAR LA PARTICULE PSEUDO-VIRALE VPL 2/6.

### A. Les animaux.

Des chèvres de race Alpine Chamoisée, nées entre 1995 et 1999 ont été utilisées dans le cadre des expériences. Elles étaient gestantes au moment de l'immunisation. 10 chèvres ont été immunisées par la particule pseudo-virale VPL 2/6 selon le protocole décrit au point C ci-après et 10 autres ont servi de témoins.

### B. Les particules pseudo-virales.

Les VLP 2/6 sont obtenues en co-infectant les cellules de *Spodoptera frugiperda 9* avec les bacculovirus recombinants BacRF2A et pAC461/SA11-6. BacRF2A, obtenu à partir de la souche bovine RF, exprime la protéine VP 2 bovine, et pAC461/SA11-6, obtenu à partir de la souche simienne SA11, exprime la protéine VP 6 (Labbé et al., 1991, *Journal of Virology*, vol. 65, pages 2946-2952). Ces VLP 2/6 sont donc formées de deux couches protéiques : la capside interne et de la couche protéique intermédiaire.

### C. Immunisation des chèvres.

Les chèvres immunisées ont reçu deux injections. La première injection a été pratiquée environ un mois avant la date de mise bas prévue (J0) et la seconde 21 jours plus tard, soit une semaine avant la date de mise bas prévue (J21). Les chèvres non immunisées (contrôle) n'ont reçu aucune injection.

Les injections ont été réalisées en préparant extemporanément l'émulsion de VLP 2/6 avec l'adjuvant de Freund incomplet.

Pour l'injection réalisée à J0 : 0,5 ml de TNC (Tris 20mM, Nacl 50mM, CaCl2 1mM pH 7,4) contenant 50 µg de VLP 2/6 et 0,5 ml de Freund incomplet ont été mis en émulsion à l'aide d'une seringue de 2 ml en aspirant et expulsant plusieurs fois le mélange dans un tube de 5 ml. Le mélange obtenu a ensuite été injecté par voie intramusculaire (IM) dans l'encolure des chèvres. Pour l'injection de rappel réalisée à J21, le même protocole a été suivi mais l'émulsion contenait 22 µg de VLP 2/6.

### I.2 IMMUNISATION DES CHEVRES PAR LA PARTICULE PSEUDO-VIRALE VPL 2/8/6/7.

### A. Les animaux.

Des chèvres de race Alpine Chamoisée, nées entre 1995 et 1999 ont été utilisées dans le cadre des expériences. Elles étaient gestantes au moment de l'immunisation. 4 lots de 5 chèvres ont été immunisés par la particule pseudo-virale VPL 2/8/6/7 selon le protocole décrit au point C ci-après et 2 autres lots de chèvres ont servi de témoins.

### B. Les particules pseudo-virales.

Les VPL 2/8/6/7 sont obtenues en co-infectant les cellules de *Spodoptera frugiperda 9* avec les bacculovirus recombinants BacRF2A, pAC461/SA11-6 et BacVP8JA16. BacRF2A, obtenu à partir de la souche bovine RF, exprime la protéine VP 2 bovine et pAC461/SA11-6, obtenu à partir de la souche simienne SA11, exprime la protéine VP 6 (Labbé et al., 1991, *Journal of Virology,* vol. 65, pages 2946-2952). BacVP8JA16, obtenu à partir de la souche RF d'un rotavirus bovin, exprime la protéine VP 8 dans son intégralité ainsi qu'un lieur et le VP2delta92.

Ces VPL 2/8/6/7 sont donc formées de trois couches protéiques : la capside interne (VPL 2), la couche protéique intermédiaire (VPL 6) et la couche externe (VPL 7 et VPL 8). Ces VPL 2/8/6/7 contiendront donc le maximum d'épitopes inducteurs d'anticorps protecteurs.

### C. Immunisation des chèvres.

Le protocole d'immunisation comprend trois immunisations aux jours 60, 90 et 115 post coïtum des chèvres gestantes, contrôlées par échographie.

La première immunisation pratiquée 60 jours après le coït (J60) consiste en l'administration par voie orale d'un vaccin réplicatif (Scourvax 2, Sanofi) destiné à stimuler l'immunité à l'IgA, notamment au niveau du tractus digectif. La seconde immunisation pratiquée 90 jours après le coït (J90) consiste en l'administration par voie intramusculaire de 100 µg de la particule pseudo-virale VPL 2/8/6/7 additionnée d'adjuvant, l'administration par voie intramusculaire permettant la diffusion de la réponse anticorps à l'ensemble de l'organisme. La troisième immunisation pratiquée 115 jours après le coït (J115) consiste en l'administration par voie intra-mammaire de 100 µg de la particule pseudo-virale VPL 2/8/6/7 additionnée d'adjuvant, l'administration par voie intra-mammaire permettant de stimuler la production d'immunoglobulines de façon optimale dans le lait. L'ensemble de ces 3 immunisations a pour but de stimuler la production de plasmocytes de façon optimale dans tout l'organisme et en particulier dans la mamelle où les plasmocytes sont orientés vers la production d'IgA. Les anticorps pourront ainsi provenir d'une production locale (mamelles) et d'une production circulante (autres organes). Un lot de chèvres non immunisées (contrôle) n'a reçu aucune injection.

Les injections ont été réalisées en préparant extemporanément l'émulsion de VPL 2/8/6/7 avec l'adjuvant. L'adjuvant ajouté est distinct selon le lot de chèvres gestantes testé.
Lot de chèvres n°1 : Adjuvant incomplet de Freund (IFA) ;
Lot de chèvres n°2 : Toxine détoxifiée de *E. coli* LT192RG (fourni par J. Clements, Tulane, USA) ;
Lot de chèvres n°3 : Adjuvant Montanide ISA 206 (SEPPIC, Air Liquide) ;
Lot de chèvres n°4 : Adjuvant Montanide ISA 206 (SEPPIC, Air Liquide) additionné de calcitriol destiné à amplifier la synthèse d'IgA.
Les différents adjuvants utilisés vont jouer un rôle déterminant sur la quantité et la qualité (IgA sécrétoires) des immunoglobulines sécrétées.

Pour l'injection réalisée à J90 : 1 ml de TNC (Tris 20mM, Nacl 50mM, CaCl2 1mM pH 7,4) contenant 100 µg de VPL 2/8/6/7 et 1 ml d'adjuvant ont été mis en émulsion à l'aide d'une seringue de 2 ml en aspirant et expulsant plusieurs fois le mélange dans un tube de 5 ml. Le mélange obtenu a ensuite été injecté par voie intramusculaire dans l'encolure des chèvres.

Pour l'injection réalisée à J115 : 1 ml de TNC (Tris 20mM, Nacl 50mM, CaCl2 1mM pH 7,4) contenant 100 µg de VPL 2/8/6/7 et 1 ml d'adjuvant ont été mis en émulsion à l'aide d'une seringue de 2 ml en aspirant et expulsant plusieurs fois le mélange dans un tube de 5 ml. Le mélange obtenu a ensuite été injecté par voie intra-mammaire dans l'encolure des chèvres.

**Tableau 1 :**

| Immunisation en 3 étapes de 6 lots de 5 chèvres gestantes par VPL 2/8/6/7 additionnée d'adjuvant. | | | | | | |
|---|---|---|---|---|---|---|
| Nombre de jours post-coït et Voie d'administration | Lot n° 1 | Lot n°2 | Lot n°3 | Lot n°4 | Lot n°5 | Lot n°6 |
| J60 Voie orale | Scourvax 2 | Scourvax 2 | Scourvax 2 | Scourvax 2 | - | Scourvax 2 |
| J90 Voie intramusculaire | VPL 2/8/6/7 - IFA | VPL 2/8/6/7 - LT192RG | VPL 2/8/6/7 - ISA206 | VPL 2/8/6/7 - ISA206 - calcitriol | - | - |
| J115 Voie intramammaire | VPL 2/8/6/7 - IFA | VPL 2/8/6/7 - LT192RG | VPL 2/8/6/7 - ISA206 | VPL 2/8/6/7 - ISA206 - calcitriol | - | - |

### II. TITRATION DES IMMUNOGLOBULINES.

### A. Méthodes.

### 1. Prélèvements.

Une prise de sang à la veine jugulaire a été réalisée sur tube EDTA, chez toutes les chèvres, à J0, J21, JMB et J36.

Pendant les 6 jours qui ont suivi la mise bas, un échantillon de colostrum de 15 ml a été prélevé sur chaque animal lors des traites du matin (échantillons référencés de C0 à C5). Les colostrums ont été ultracentrifugés sur une centrifugeuse Beckman L8-70M avec un rotor 50.2 à angle fixe, pendant une heure à 35000 tours par minute, à 5°C. Suite à cette ultracentrifugation, le lactosérum a été séparé des lipides et stocké dans des tubes Eppendorf de 1 ml à -20°C, en vue des dosages ultérieurs.

### 2. Méthodes de dosage et calcul des titres.

Les dosages ont été effectués par la méthode ELISA (Enzyme Linked ImmunoSorbent Assay).

### a. Dosage des IgG.

Le même protocole a été utilisé pour doser les IgG dans les plasmas et dans les lactosérums.

Ainsi, les puits de plaques Dynatech de 96 puits ont été "coatés" avec 0,1 µg de VLP 2/6 diluées dans 100 µl de tampon bicarbonate de sodium 0,1M pH=8,3. Les plaques ont alors été placées une nuit à 4°C. Elles ont été lavées 5 fois avec du PBS-Tween 20 (0,05%). Puis une saturation avec du PBS-SVF 5% (PBS contenant 5% de sérum de veau foetal) pendant 1h à 37°C a été réalisée. Les plaques ont été lavées une fois.

Les échantillons de sérum des animaux immunisés ont été dilués dans du PBS-SVF 5%, du 1/1000^{ème} au 1/243000^{ème} (voir jusqu'au 1/19000000^{ème} pour certains). Par ailleurs, pour certains échantillons contenant peu d'IgG anti-rotavirus, des dilutions sériées de raison 3 du 1/5 au 1/1200 ont été réalisées. 100 µl de chaque dilution sont déposés en duplicate. Sur chaque plaque, les puits ne contenant pas d'échantillon ont reçu 100 µl de PBS-SVF 5%, ils servent de témoins négatifs. Un plasma de chèvres immunisées vis-à-vis du rotavirus, lors d'expériences antérieures, a servi de témoin positif. Après une heure d'incubation à température ambiante, les puits ont été lavés 6 fois avec du PBS-Tween 20.

Puis 100 µl de PBS-SVF 5%, contenant un anticorps anti-IgG de chèvre (dilué au 1/4000^{ème}), développé chez le lapin, et couplé à la peroxydase ont été déposés dans chaque puits. Après une heure d'incubation à température ambiante, les plaques ont été lavées 5 fois au PBS-Tween 20.

Enfin, 100 µl du substrat de la peroxydase (1/4 TMB + 1/4 H₂O₂+ 1/2 H₂O) ont été déposés dans chaque puits. Après 7 minutes à température ambiante, le substrat est retiré et la réaction arrêtée en distribuant 50 µl de H₃PO₄ à 2M dans chaque puits. La lecture de la densité optique (DO) a été réalisée à une longueur d'onde de 450 nm (Multiscan).

### b. Dosage des IgA dans les lactosérums.

Les différences par rapport au protocole présenté en (a.) sont les suivantes : les plaques ont été coatées avec 0,2 µg de VLP 2/6 ou VPL 2/8/6/7 par puits et les échantillons de colostrum ont été dilués au 1/10^{ème}, puis au 1/100^{ème}, puis par dilutions sériées de raison 3 (1/300, 1/900, 1/2700, 1/8100). L'anticorps secondaire utilisé était un anti-IgA de chèvre, couplé peroxydase, produit chez le lapin et fourni par le laboratoire Interchim (A50-106P).

### c. Calcul du titre.

Le titre est calculé en réalisant des dosages ELISA sur différentes dilutions successives de l'échantillon puis en traçant la courbe DO=f(log(dilution de l'échantillon)) où DO est la Densité Optique donnée par le dosage ELISA. Les dilutions sont réalisées jusqu'à avoir une DO inférieure au double du bruit de fond. Une lecture graphique, sur la courbe tracée précédemment, permet de déterminer la dilution qui donne une DO double de celle du bruit de fond ; c'est la dernière dilution qui donne un signal positif. L'opposé du logarithme en base 10 de cette dernière dilution constitue le titre.

Les résultats fournis par la suite sont les moyennes arithmétiques des titres calculés selon cette méthode.

### d. Analyse statistique.

Le test statistique employé pour comparer les moyennes est un test de Mann et Withney bilatéral. Les calculs ont été effectués avec les logiciels Microsoft Exce12000® et WinSPAD 5.0®. La filière Xlstat de WinSPAD a été utilisée. La différence a été considérée comme significative si la P-value du test de Mann et Withney était inférieure à 2,5% (α=5%).

### B. Résultats.

### 1. Réponse sérique en IgG.

Les résultats sont présentés dans le tableau 2 ci-dessous. A J0, les titres en IgG spécifiques anti-rotavirus sont identiques chez les animaux immunisés et chez les animaux non immunisés. A J21, le titre moyen des animaux immunisés a augmenté d'au moins une unité logarithmique par rapport à ce qu'il était à J0. Le titre moyen des animaux non immunisés n'a pas augmenté. Entre J21 (jour de l'injection de rappel) et le jour de la mise bas (MB) ou J28, le titre moyen des animaux immunisés a peu augmenté.

**Tableau 2 :**

| Moyennes et écarts-types des titres sériques en IgG des chèvres immunisées et des chèvres non immunisées. | | | | | |
|---|---|---|---|---|---|
| **Groupes** | **J0** | **J21** | **J28** | **MB** | **J36** |
| **Immunisées** | 3,99 | 5,07 | 5,18 | 5,20 | 5,4 |
| **Non immunisées** | 3,92 | 3,81 | 3,90 | 3,92 | 3,9 |
| σ **immunisées** | 0,54 | 0,43 | 0,48 | 0,48 | 0,4 |
| **σ non immunisées** | 0,30 | 0,46 | 0,37 | 0,36 | 0,4 |
| **p(MW)** | 0,381000 | 0,000188 | 0,000162 | 0,000164 | 0,000078 |

σ=écart-type. p(MW)=P-value du test de Mann et Withney.

En conclusion, les chèvres en élevage possèdent déjà des anticorps anti-rotavirus, le titre ne change pas chez les chèvres non immunisées au cours du mois qui précède la mise bas et l'immunisation par les VLP 2/6 a été efficace. Elle permet d'obtenir une augmentation d'au moins une unité logarithmique en moyenne. Après le rappel, l'augmentation de titre est peu marquée. Le rappel ne semble donc pas nécessaire pour augmenter encore le titre.

### 2. Réponse colostrale en IgG.

Les résultats sont présentés dans le tableau 3 ci-après. Le titre colostral anti-rotavirus moyen est plus élevé chez les chèvres immunisées que chez les chèvres non immunisées quel que soit le jour après la mise bas. Chez les animaux non immunisés, le titre moyen diminue rapidement entre C0 (jour de la mise bas) et C5 : deux unités logarithmiques sont perdues (de 4,42 à C0 pour finir à 2,65 à C5). Chez les animaux immunisés, le titre diminue également entre C0 et C5, mais seulement d'une unité logarithmique. De plus, le titre moyen se maintient à une valeur élevée jusqu'à C5 (4,72). Au final, le titre moyen à C5 chez les animaux immunisés est supérieur de 2 unités logarithmiques au titre moyen à C5 chez les animaux non immunisés.

Ainsi, les VLP 2/6 (50 µg puis 22 µg 21 jours plus tard), administrées avec de l'adjuvant de Freund incomplet, par voie intramusculaire, induisent une réponse immune colostrale en IgG spécifique anti-rotavirus. La réponse induite se caractérise par un titre en IgG plus élevé le jour de la mise bas et par une persistance de ce titre à un niveau plus élevé pendant au moins les 5 premiers jours de la période colostrale.

**Tableau 3 :**

| Moyennes et écarts-types des titres colostraux en IgG des chèvres immunisées et des chèvres non immunisées. | | | | | | |
|---|---|---|---|---|---|---|
| **Groupe** | **C0** | **C1** | **C2** | **C3** | **C4** | **C5** |
| **Immunisées** | 5,87 | 5,36 | 5,19 | 5,02 | 4,97 | 4,7 |
| **Non immunisées** | 4,42 | 3,79 | 3,49 | 2,96 | 2,76 | 2,6 |
| **σ immunisées** | 0,40 | 0,36 | 0,24 | 0,40 | 0,35 | 0,4 |
| **σ non immunisées** | 0,33 | 0,43 | 0,64 | 0,57 | 0,66 | 0,6 |
| **p(MW)** | 0,000564 | 0,000316 | 0,000313 | 0,000313 | 0,000316 | 0,000311 |

σ=écart-type. p(MW)=P-value du test de Mann et Withney.

### 3. Réponse colostrale en IgA.

### 3.1. Réponse colostrale en IgA consécutive à l'immunisation par la particule pseudo-virale VPL 2/6.

Les résultats sont présentés dans et le tableau 4 ci-après. De C0 à C5, les différences de titre entre chèvres immunisées et chèvres non immunisées sont significatives. Les chèvres immunisées ont un titre en IgA supérieur de 0,75 à 1,2 unités logarithmiques par rapport aux chèvres non immunisées.

Les VLP 2/6 (50 µg puis 22 µg 21 jours plus tard), administrées avec de l'adjuvant de Freund incomplet, par voie intramusculaire, induisent une réponse immune colostrale en IgA spécifique anti-rotavirus.

Chez les animaux immunisés, le titre en IgA persiste à un niveau élevé pendant au moins les 5 premiers jours de la période colostrale.

**Tableau 4 :**

| Moyennes et écarts-types des titres colostraux en IgA des chèvres immunisées et des chèvres non immunisées. | | | | | | |
|---|---|---|---|---|---|---|
| **Groupe** | **C0** | **C1** | **C2** | **C3** | **C4** | **C5** |
| **Immunisées** | 3,47 | 3,55 | 3,07 | 2,63 | 2,46 | 2,5 |
| **Non immunisées** | 2,73 | 2,27 | 1,82 | 1,62 | 1,67 | 1,6 |
| **σ immunisées** | 1,21 | 0,62 | 0,68 | 0,65 | 0,57 | 0,4 |
| **σ non immunisées** | 0,44 | 0,46 | 0,30 | 0,41 | 0,35 | 0,3 |
| **p(MW)** | 0,02 | 0,001 | 0,0010 | 0,002 | 0,003 | 0,001 |

σ=écart-type. p(MW)=P-value du test de Mann et Withney.

### 3.2. Réponse colostrale en IgA consécutive à l'immunisation par la particule pseudo-virale VPL 2/8/6/7.

Les résultats sont présentés dans et le tableau 5 ci-après. De C0 à C15, les différences de titre entre chèvres immunisées et chèvres non immunisées sont significatives. Les chèvres immunisées (lots n° 1 à 4) ont un titre en IgA supérieur de 0,5 à 1,2 unités logarithmiques par rapport aux chèvres non immunisées (lots n°5).

Les chèvres de lots n° 1 à 4 immunisées par les 3 étapes d'immunisation successives (Scourvax 2 par voie orale à J60 post-coït ; 100 µg de VPL 2/8/6/7 par voie intramusculaire à J90 post-coït ; puis 100 µg de VPL 2/8/6/7 par voie intra-mammaire à J115 post-coït) ont un titre en IgA supérieur de 0,5 à 1,2 unités logarithmiques par rapport au lot de chèvres n°6 immunisées par administration de Scourvax 2 par voie orale à J60 post-coït.

Entre C0 et C10, la réponse immune colostrale en IgA spécifique anti-rotavirus observée induite par les VPL 2/8/6/7 administrées avec de l'adjuvant ISA206 (lot n°3) est légèrement supérieure à celle induite par les VPL 2/8/6/7 administrées avec les autres types d'adjuvant (lot n° 1, 2 et 4).

A C15, la réponse immune colostrale en IgA spécifique anti-rotavirus observée induite par les VPL 2/8/6/7 administrées avec de l'adjuvant ISA206 (lot n°3) et celle induite par les VPL 2/8/6/7 administrées avec l'adjuvant ISA206 additionné de calcitriol (lot n°4) est sensiblement identique. Par ailleurs, à C15, la réponse immune colostrale en IgA spécifique anti-rotavirus observée induite par les VPL 2/8/6/7 administrées avec de l'adjuvant ISA206 (lot n°3) ou l'adjuvant ISA206 additionné de calcitriol (lot n°4) est légèrement supérieure à celle induite par les VPL 2/8/6/7 administrées avec un adjuvant de type IFA ou LT192RG (lot n°1 et 2).

Chez les animaux immunisés (lots n° 1 à 4), le titre en IgA persiste à un niveau élevé pendant les 15 premiers jours de la période colostrale.

Au terme de cette étude, il s'avère que les protocoles de vaccination ont des effets quasiment équivalents sur les titres en IgA anti-rotavirus. L'ensemble des injections stimule la production de plasmocytes orientés vers la production d'IgA dans l'organisme et proviennent donc d'une production locale et circulante. Les schémas de vaccination étudiés présentent tous un résultat au moins équivalent à celui mettant en oeuvre le vaccin en adjuvant Freund. L'injection des VLP 2-8/6/7 avec adjuvant Montamide ISA 206 (lot 3 et 4) semble apporter la meilleure réponse, sans que le bénéfice de l'addition de calcitriol destiné à amplifier la synthèse d'IgA soit clairement mis en évidence. De même, le bénéfice de l'injection intra-mammaire n'est pas clairement mis en évidence, et tout laisse à penser qu'une seule injection intramusculaire doit être recommandée.

**Tableau 5 :**

| Moyennes des titres colostraux en IgA des chèvres immunisées et des chèvres non immunisées. | | | | |
|---|---|---|---|---|
| Lot de chèvres | C0 | C5 | C10 | C15 |
| Lot n°1 (VPL 2/8/6/7 - IFA) | 3,47 | 2,5 | 2,31 | 2,44 |
| Lot n°2 (VPL 2/8/6/7 - LT192RG) | 3,55 | 2,62 | 2,28 | 2,31 |
| Lot n°3 (VPL 2/8/6/7 - ISA206) | 3,89 | 2,81 | 2,51 | 2,50 |
| Lot n°4 (VPL 2/8/6/7 - ISA206 - calcitriol) | 3,61 | 2,59 | 2,22 | 2,51 |
| Lot n°5 (non immunisé) | 2,49 | 2,01 | 1,50 | 1,39 |
| Lot n°6 (témoin Scourvax 2) | 2,85 | 2,33 | 1,95 | 1,68 |

### III. EXTRACTION DES IMMUNOGLOBULINES COLOSTRALES ANTI-ROTAVIRUS.

Le colostrum contient des lipides, des caséines (40mg/ml), de la β-lactoglobuline (30mg/ml), de l'α-lactalbumine, de la sérum albumine et des immunoglobulines (80mg/ml). Afin d'obtenir un produit concentré en immunoglobulines, qui soit facile à conserver, il faut traiter le colostrum de façon à en extraire les immunoglobulines et le déshydrater. Pour purifier les immunoglobulines, nous avons appliqué une méthode proposée par Grongnet al. en 1996 (Lait, vol. 76, pages 303-309). Deux méthodes de déshydratation ont été comparées : la lyophilisation et l'atomisation.

L'efficacité des étapes de purification des immunoglobulines a été évaluée par électrophorèse et la persistance des immunoglobulines spécifiques anti-rotavirus a été évaluée au cours du processus de purification en réalisant des dosages ELISA sur des échantillons prélevés aux différentes étapes.

### A. Matériel et méthodes.

### 1. Procédé de séparation des immunoglobulines colostrales.

L'essai en laboratoire a été réalisé avec sur 5 litres de colostrum provenant d'une chèvre immunisée vis-à-vis du rotavirus (titre moyen du colostrum = 5,14). La figure 1 représente les différentes étapes du procédé. Après avoir été décongelé, le colostrum a été dilué et écrémé. Le pH du produit écrémé a été ensuite abaissé par ajout d'HCl, afin de précipiter les caséines. Le pH a ensuite été augmenté par ajout de soude et le produit a été centrifugé. Le jus de centrifugation a été filtré (noté 1) avant de subir une ultrafiltration sur un filtre de porosité 50 kD (module minéral Kerasep). Cette étape permet de séparer les protéines selon leur taille : les protéines ayant un PM supérieur à 50 kD sont retenues dans le rétentat d'ultrafiltration (noté 3) et celles de moins de 50 kD passent dans le perméat d'ultrafiltration (noté 2).

Une partie du rétentat a été atomisée (noté 4). L'atomisation est un procédé de déshydratation. Le rétentat est injecté sous pression sous forme de fines gouttelettes dans un courant d'air chaud (180 °c) et sec, ce qui provoque l'évaporation de l'eau. Le produit final est une poudre. L'atomisation est effectuée après ajout de 20% de maltodextrine, ceci afin d'éviter le collage du produit dans la tour d'atomisation et toute dégradation thermique. L'autre partie du rétentat a été lyophilisée (noté 5). La lyophilisation est un procédé de déshydratation à froid qui utilise le principe de la sublimation : sous l'effet du vide, la glace contenue dans le produit s'évapore. Les dosages protéiques et ELISA ont été réalisés sur des échantillons prélevés aux étapes notées 1, 2, 3, 4 et 5 de la figure 1.

### 2. Contrôle de la purification :.

Pour chaque étape numérotée de 1 à 5, un dosage protéique, une électrophorèse et des dosages par méthode ELISA ont été réalisés et ce, afin de vérifier la purification des IgG anti-rotavirus et la conservation de leur activité *in vitro.*

### a. Remise en solution des phases solides.

Les rétentats atomisés et lyophilisés étaient en phase solide. Pour réaliser les dosages ELISA, il a fallu les réhydrater de façon à les ramener au taux de MS de l'étape précédant le séchage (le rétentat), c'est-à-dire à 1,72% de MS. 22 mg de rétentat atomisé ont été dilués dans 1 ml d'eau et 18 mg de rétentat lyophilisé dans 1 ml d'eau.

### b. Dosage protéique au cours des différentes étapes.

Afin de suivre la capacité du procédé à extraire les protéines colostrales, un dosage protéique par la méthode BCA (Interchim) a été effectué sur les étapes numérotées de 1 à 5.

### c. Electrophorèse.

Nous avons réalisé une séparation des protéines, en conditions dénaturantes, sur gel SDS-PAGE (Resolving gel 12% et stacking gel 5%). Les chaînes lourdes et légères des immunoglobulines sont séparées. Le marqueur de taille (Dalton Mark VII L) de Sigma a été utilisé. 20 ou 10 µg de protéines ont été déposées par piste. La coloration a été réalisée avec le bleu de Coomassie. Le gel a ensuite été séché sous vide.

Une autre séparation a été réalisée en conditions non dénaturantes sur gel SDS-PAGE (resolving gel 8% et stacking gel 5%). Le marqueur de taille (Stock No SDS -6H) de Sigma a été utilisé.

### d. Tests ELISA.

Les IgG anti-rotavirus ont été dosées par méthode ELISA.

### d.1. Dosage des IgG totales : évaluation du titre des différentes fractions purifiées.

Les échantillons testés correspondent à 100 µl des différentes fractions. Le protocole ELISA et le calcul des titres ont été faits comme décrit précédemment. Le titre permet d'appréhender directement la concentration en IgG anti-rotavirus dans les différents produits. Plus le titre est élevé plus la concentration en IgG anti-rotavirus est élevée.

### d.2. Évaluation in vitro de la pureté en IgG anti-rotavirus des produits et de la perte d'activité des IgG anti-rotavirus.

Au cours du processus de purification, le colostrum et les produits obtenus ont subi des étapes de dilution, de concentration et un séchage final. Les dosages ELISA ont été réalisés après avoir ramené chaque échantillon (de 1 à 5) à une quantité de protéines totales identiques. De cette manière, les DO obtenues à l'issue du test ELISA reflètent la pureté protéique en IgG spécifiques anti-rotavirus : plus la DO est élevée pour une même quantité de protéines plus il y a d'IgG anti-rotavirus au sein de ces protéines. Cela permet également d'estimer la dégradation des IgG anti-rotavirus suite aux étapes de séchage.

Des solutions de même concentration protéique à partir des dosages protéiques réalisés par BCA ont été préparées et un échantillon contenant 0,33 µg de protéines a été déposé dans les puits des plaques ELISA. La suite du dosage ELISA a été réalisée comme décrit précédemment.

### B. Résultats : contrôle de la purification.

### 1. Dosages protéiques dans les différentes fractions.

Avant l'ultrafiltration, la concentration en protéine est de 2,33 mg/ml. Après l'ultrafiltration, elle est de 6,24 mg/ml dans le rétentat et de 0,38 mg/ml seulement dans le perméat (tableau X page 51). Sur les 19 g de protéines présents avant l'ultrafiltration, 13 g sont retrouvés dans le rétentat, c'est-à-dire 70%. L'ultrafiltration permet donc de concentrer les protéines et de se débarrasser de la MS contenant peu de protéines. Ainsi, 60 % de la MS se trouve dans le perméat.

### 2. Contrôle par électrophorèse.

Sur le gel SDS-PAGE en conditions non dénaturantes (données non montrées), il a été vérifié que la baisse de pH à 4,6 puis la centrifugation ont permis d'éliminer les caséines.

De plus, l'ultrafiltration à 50kD retient les immunoglobulines mais n'élimine pas les petites protéines.

### 3. Tests ELISA : devenir des IgG anti-rotavirus.

### a. Les dosages ELISA confirment la concentration des immunoglobulines anti-rotavirus par l'ultrafiltration.

Le titre calculé pour le perméat d'ultrafiltration est faible (1,6). De plus la DO mesurée dans le perméat d'ultrafiltration pour une quantité de 0,33 µg de protéines est nulle. Les protéines contenues dans le perméat ne sont donc pas des immunoglobulines anti-rotavirus.

Le titre dans le rétentat est de 4,7. Il était de 4,2 avant l'ultrafiltration. Les IgG anti-rotavirus sont donc plus concentrées après cette étape.

### b. Atomisation et lyophilisation provoquent toutes deux une perte modérée d'activité des anticorps anti-rotavirus.

Les titres calculés après atomisation et lyophilisation sont identiques. Ils sont légèrement inférieurs au titre calculé pour le rétentat. La DO mesurée pour 0,33 µg de protéines est de 0,91 dans le rétentat. Elle est de 0,62 après atomisation et de 0,76 après lyophilisation.

Ainsi, l'atomisation et la lyophilisation entraînent toutes deux une perte d'activité des IgG anti-rotavirus. Cette perte est modérée. La différence entre atomisation et lyophilisation est nulle en terme de titre. Par contre, pour une quantité de protéine donnée la lyophilisation dégrade moins les IgG anti-rotavirus que l'atomisation.

## Revendications

1. Composition à administration orale chez l'homme comprenant des immunoglobulines spécifiques du rotavirus produites chez un mammifère immunisé avec des pseudo particules virales.

2. Composition selon la revendication 1, **caractérisée en ce que** lesdites pseudo particules virales sont soit des pseudo particules virales homologues, soit des pseudo particules virales hétérologues.

3. Composition selon l'une des revendications 1 ou 2, **caractérisée en ce que** lesdites pseudo particules virales contiennent plusieurs protéines structurales différentes.

4. Composition selon l'une quelconque des revendications précédentes, **caractérisée en ce qu'**une desdites protéines structurales est la VP2.

5. Composition selon l'une quelconque des revendications précédentes, **caractérisée en ce que** lesdites particules pseudo virales sont choisies dans le groupe comprenant VLP 2/6, VLP 2/7, VLP 2/4/6, VLP 2/6/7 et VLP 2/4/6/7 et VLP 2/8/6/7.

6. Composition selon l'une quelconque des revendications précédentes, **caractérisée en ce que** les immunoglobulines présentes dans la composition de l'invention sont des immunoglobulines A.

7. Composition selon l'une quelconque des revendications précédentes, **caractérisée en ce que** lesdites immunoglobulines sont obtenues à partir du colostrum produit par ledit mammifère.

8. Composition selon l'une quelconque des revendications précédentes, **caractérisée en ce que** lesdites immunoglobulines sont présentes sous la forme d'une fraction semi-purifiée du colostrum.

9. Composition selon l'une quelconque des revendications précédentes, **caractérisée en ce que** ledit mammifère est choisi dans le groupe constitué par la vache, la brebis, la chèvre, la lapine et la truie.

10. Composition selon l'une quelconque des revendications précédentes, **caractérisée en ce que** ledit mammifère est un ruminant laitier et, de préférence, la chèvre.

11. Composition selon l'une quelconque des revendications précédentes, utile pour la protection passive contre les pathologies induites par les rotavirus.

12. Utilisation d'une composition selon l'une quelconque des revendications précédentes en tant que complément alimentaire.

13. Procédé de préparation d'une composition selon l'une quelconque des revendications 1 à 11, comprenant les étapes suivantes :
a) préparation des pseudo particules virales,
b) immunisation d'un mammifère avec les pseudo particules virales obtenues à l'étape (a),
c) récupération du colostrum produit par ledit mammifère,
d) éventuellement concentration des immunoglobulines contenues dans le colostrum récupéré à l'étape (c).

14. Procédé de préparation selon la revendication 13, **caractérisé en ce que** l'immunisation de l'étape (b) est une immunisation par voie intramusculaire, par voie intra-nasale, par voie intra-vaginale ou per os.

15. Procédé de préparation selon l'une quelconque des revendications 13 ou 14, **caractérisé en ce que** la concentration des immunoglobulines à l'étape (d) comprend au moins une étape choisie dans le groupe constitué par un écrémage du colostrum, une précipitation des caséines, des centrifugations, des filtrations, des ultrafiltrations, une lyophilisation et une atomisation.
